# EUROPEAN PATENT APPLICATION

(11) **EP 4 527 879 A1**
(43) Date of publication of application: **26.03.2025**
(21) Application number: 23198393.3
(22) Date of filing: 19.09.2023
(51) Int. Cl.: C08J 11/14, C08J 11/16

(54) **PROCESS FOR RECOVERING CHEMICALS IN A PROCESS FOR RECYCLING BLEND TEXTILES**

(71) Applicant: Sanko Tekstil Isletmeleri San. Ve Tic. A.S., 16400 Inegol - Bursa (TR)
(72) Inventor: KONUKOGLU, Fatih, 16400 Inegol - BURSA (TR); KAPLAN, Gökhan, 16400 Inegol - BURSA (TR); ÖKTEM, Gözde, 16400 Inegol - BURSA (TR); HAMITBEYLI, Agamirze, 16400 Inegol - BURSA (TR); LOYAN, Kenan, 16400 Inegol - BURSA (TR); AKBULUT, Sabrettin, 16400 Inegol - BURSA (TR); ZENGIN, Nuriye, 16400 Inegol - BURSA (TR)
(74) Representative: Gislon, Gabriele

(57) **Abstract**

Process to recover NaOH, polyester monomers, dyes and byproducts from an alkaline depolymerization liquor resulting from a depolymerization reaction of polyester in a blend textile. The process comprises concentrating the liquor solution in a mixture comprising water, solid NaOH, dyes and other liquid products, extracting ethylene glycol from the mixture, dissolving solid NaOH, recovering solid dyes and recovering terephthalic acid.

## Description

### Field of the invention

The present invention relates to a process for recovering chemicals, i.e. chemical products including dyes, obtained in a process for recycling blend textiles. In greater detail, the present invention relates to a process for recovering and isolating NaOH, polyester monomers and dyes obtainable in a process for recycling polyester blend fabrics through alkaline depolymerization of the polyester. The invention's process is particularly suitable to those recycling processes providing for recovering and recycling cellulosic staple fibers from fabrics containing polyester filaments or fibers and cellulosic staple fibers, particularly cotton staple fibers. The invention also relates to a process of recovering vat dyes from a textile.

### State of the art

The textile industry has a huge impact on the environment due to large use of petrochemical and natural resources and to the present trend that increases the consumption of garments while reducing their life-span. Recycling waste textiles from fabrics, used garments apparels and similar has become an important issue in fashion field. The cost of recycling waste textile includes not only the retrieving of waste textile but also to define proper methods for treatment waste textile which can be financially sustainable on a large scale. Polyester, e.g. polyethylene terephthalate (PET) fibers, and cotton are the most widely used man-made and natural fibers. Known methods for treatment of waste textile include steps to remove polyester from the waste textile by depolymerization and to recover the products of depolymerization to be further processed, e.g. to again obtain polyester which can be reused.

WO2022195433A1 describes a method for recycling a polycotton waste textile, i.e. a textile comprising a cotton component and a polyester component, where the textile is submitted to a depolymerization reaction of the polyester component by basic hydrolysis such as in sodium hydroxide solution. The recovery of terephthalic acid is carried out through a step of terephthalic acid precipitation, terephthalic acid separation, terephthalic acid washing, crystallization and terephthalic acid drying.

Patent application EP23156251, in the name of the present applicant, filed 13.02.2023 and here incorporated by reference, discloses a process for recycling polycotton textiles, in particular fabrics and garments, containing polyester filaments or fibers and cellulosic staple fibers, particularly cotton staple fibers.

The process of EP'251 comprises a depolymerization step of polyester with an alkaline depolymerizing solution that results in the degradation of polyester into monomers and in a structure of cellulosic fibers that is substantially corresponding to the structure of the original polycotton fabric, minus the polyester portion of the same. A step of bleaching the treated textile with a bleaching solution after the depolymerization of the polyester and a step of washing the treated textile with a washing solution after bleaching the treated textile are also provided for.

In particular the invention of EP'251 relates to a process for recycling waste blend textiles comprising polyester and cellulosic fibers, preferably cotton fibres, the process including a depolymerization of the polyester in a basic aqueous solution, comprising the following steps:
a) providing an amount of said textiles in a reaction chamber;
b) providing an amount of said basic depolymerization solution according to a bath ratio in the range of 1/2 and 1/20 by weight of textile over weight of solution;
c) circulating said amount of depolymerizing solution through said textiles to depolymerize said polyester into corresponding monomers and to remove said polyester monomers from said textiles;
d) removing said depolymerizing solution from said reaction chamber;
   wherein the temperature of the said depolymerizing solution in step c) is in the range between 101 °C and 160 °C, preferably between 120° to 160°C, more preferably between 130°C to 140°C.
   and wherein
e) the textiles are in a still condition and the solution is circulated through them to provide treated textiles comprising cellulosic staple fibers, preferably cotton staple fibres, substantially free from polyester material.
   The reaction is carried out for a time between 100 minutes and 150 minutes, more preferably 120 minutes. The depolymerization solution is pumped through the textile in the reactor, the textile being held in a still, i.e. static condition.

In other words, in the process of EP'251 the chamber is fed with a depolymerizing solution to depolymerize polyester fibres of the waste blend textiles maintained in a still condition within the reaction chamber under reaction conditions allowing the cleavage of the ester bonds of polyester into its monomers, terephthalic acid as disodium salt (Na₂TP), and ethylene glycol (EG), resulting in a substantial detachment or removal of dyes, particularly indigo and other vat dyes, from the fabric and dramatically reducing the depolymerization of the cellulosic staple fibres of the fabric.

The depolymerizing solution, or reaction liquor, as well as the bleaching solution and the washing solution, contain chemicals such as NaOH, TPA, Na₂TP, EG, dyes such as indigo dyes, reactive dyes, sulphur dyes.

Said solutions (depolymerization solution, bleaching solution, washing solution) can be reused multiple times in the process whereby the concentration of chemicals (namely Na₂TPA, EG, and dyes) is increased after each cycle. The applicant has realized that increased number of cycles has a positive effect on the processability of the solutions to recover the chemicals mentioned above, including the dyes.

Furthermore, the applicant has realized that if the process of recovering the chemicals is performed with the mentioned solutions (depolymerization solution, bleaching solution, washing solution) after multiple cycles, the efficiency of recovering chemicals increases as more compounds to be treated are present in the solutions. The efficiency can be in term of reduced process costs, labour costs and energy consumption.

A common problem in recycling PET fibers from polycotton textiles and specifically from garments and fabrics is the presence of further products and especially the presence of some amounts of dyes together with the monomers TPA and EG in the depolymerization reaction mixtures.

Another problem is to recover NaOH from the reaction mixture, or reaction liquor, to be reused in further processes, particularly of the same recycling cycle.

It is thus an aim of the present invention to solve the above-mentioned drawbacks of the known processes of recycling polyester-cotton textiles and to provide a method to increase the purity of the polyester monomers recovered from the depolymerizing solution and from the other solutions and mixtures resulting from the known textile recycling depolymerization processes.

Another aim of the present invention is to recover NaOH, polyester monomers and indigo or indigo type dyes present in the depolymerizing solution (and also in the bleaching solution and in the washing solutions) as obtainable with the process of EP23156251. The recover process may be carried out either separately on the different solutions or after the depolymerizing solution, the bleaching solution and the washing solutions have been collected in a single solution to be treated.

### SUMMARY OF THE INVENTION

The above aims are reached by means of the process according to claim 1 of the present description. Preferred embodiments are recited in the dependent claims. A further object of the invention is a process of recovering vat dyes from a textile, according to claim 12.

The starting material for the process of the invention is the mixture resulting from a process in which the polyester part of a textile is depolymerized using a depolymerization solution containing NaOH. After the depolymerization process, the monomers terephthalic acid (TPA) and ethylene glycol (EG) are formed. In addition, the solution also contains dyes, particularly vat dyes, namely indigo and indigoid dyes or indigo type dyes, that were present on the cotton or cellulosic part of the textile and that have been removed from the surface of the textile and remain in the depolymerization solution. In some cases, other widely used polyesters such as polypropylene terephthalate (PPT), polytrimethylene terephthalate (PTT), polybutylene terephthalate (PBT), and the like, or mixtures of these polyesters, may be present; in this case the polyesters would have been hydrolysed by the process to the corresponding monomers, i.e. Na₂TPA and butane diol, propylene diol, trimethylene glycol.

The term "textiles" is here referred to fabrics, garments, yarns and fibers. The fabric may be a woven fabric, a knitted fabric or a non-woven fabric. Garments are encompassing any apparel item such as T-shirts, trousers, jeans, jackets, shirts, sport garments etcetera. Exemplary polycotton yarns are corespun yarns having a polyester core and a cotton sheath including cotton staple fibers. Blend non-woven textiles may include non-woven polyester filaments on a cotton or cellulosic substrate.

The term "cellulosic fibres" is here defining cotton fibers, cotton derivative fibers, fibers of modified cotton and other fibres typically used in a yarn such as linen fibres, fiber flax, jute fibres, hemp fibres, ramie fibres, kenaf fibres, sisal fibres, henequen fibres and similar. The cotton and cellulosic fibers are generally in the form of staple fibers in a yarn. Viscose and other man-made cellulose-based fibers and filaments fall under the definition of cellulosic fibers in this application. In both cases the cotton fibers or cellulosic fibers are recovered as such (e.g. in the original form of staple fibers of a yarn) rather than being reduced to a pulp.

The term "depolymerization solution" or "depolymerization liquor" (DL) is here defining an aqueous mixture (or aqueous liquor) that contains NaOH to reach a pH of about 13, (with an average NaOH concentration of about 3% by weight) and the monomers resulting from the alkaline depolymerization reaction of the polyester fibres of the waste textile. The said DL contains the disodium salt of terephthalic acid (Na₂TPA) and ethylene glycol (EG). Because the waste textiles derive from dyed fabrics or garments, the DL may also contain indigo and indigoid dyes that detached from the textiles e.g. by the mechanical action of the depolymerizing solution on the textiles, and may also contain cellulose fibers and other reaction products. Other dyes possibly present in the DL are hydrolysed reactive dyes, sulphur dyes etcetera.

In an aspect, the invention provides to change the pH of the solution to recover Na₂TPA by selectively modifying the alkaline pH of the depolymerization liquor to change the physical status of Na₂TPA between dissolved salt in an aqueous solution and solid salt. Liquid impurities are removed from solid salt and solid impurities are removed from Na₂TPA as dissolved salt in aqueous solution.

In another aspect, the invention relates to a process of recovering NaOH from a depolymerization liquor to be used for further depolymerization processes of blend textiles, by precipitation of Na₂TPA as a solid salt from an aqueous solution containing NaOH and Na₂TPA (in a dissolved state).

The invention process comprises the steps of providing an aqueous alkaline depolymerization liquor, typically at a pH that is about 13; concentrating said liquor to a lutarious product (i.e. a mud-like mixture, with a solid content in the range of 40% to 80% by weight) that is a mixture comprising water, solid NaOH and the solid and liquid products (including dyes) of the depolymerization reaction; extracting EG, or other glycols, from said lutarious mixture with an organic solvent; modifying the pH of the undissolved solid phase remaining from the extraction of EG to first dissolve Na₂TPA and subsequently precipitate TPA as a sodium salt or as an acid. Vat dyes, particularly indigo and indigoids, are recovered from the said undissolved solid phase. Alternatively, sulphur dyes may be recovered with the invention process. Disperse dyes present in the depolymerizing liquor might typically be dissolved in the liquid organic phase.

According to an aspect of the invention, the process of the invention comprises the following steps:
a) evaporating part of the water from the depolymerization liquor to provide a concentrated mixture containing reaction products of depolymerization;
b) extracting the said mixture with an organic solvent to obtain a liquid phase containing EG, water and NaOH and a solid phase containing NaOH, Na₂TPA, water, optionally dyes;
c) dissolving at least the Na₂TPA of said solid portion in an alkaline solution having Ph in the range of 9.5 to 11.0 whereby said solution comprises a solid content including dyes;
d) separating said solid content including dyes from said alkaline solution to obtain a purified alkaline solution and solid dyes;
e) precipitating TPA from said purified alkaline solution;
f) recovering EG and NaOH from said liquid phase.

According to an aspect, aqueous depolymerization liquor is concentrated to obtain a lutarious solid, i.e. a concentrated mixture having a mud consistency, under vacuum at a temperature below 100°C until the solids content of the resulting concentrated mixture is in the range of 40% to 80% by weight, e.g about 50% to 65%, by weight. Advantageously, the water evaporated in this step can be reused in the process, e.g. it is added to the lutarious concentrated mixture after the step of extraction with organic solvent, in order to dissolve NaOH and Na₂TPA in an alkaline solution where dyes are still present as solid particles that can be removed from the solution.

In an embodiment, the purified alkaline solution is added with NaOH to precipitate Na₂TPA; NaOH is preferably added to the said solution as a concentrated solution up to 20% by weight, to provide a NaOH solution suitable for being used in the depolymerization process of blend fabrics and textiles. A suitable NaOH solution for the process according to EP'251 is an NaOH solution at 48 Baumè.

It is thus an advantage of this step of alkaline precipitation of Na₂TPA, that after removing the disodium salt of terephthalic acid (Na₂TPA) from the alkaline solution in a way known per se, e.g. by filtration or centrifugation, the remaining alkaline solution is suitable to be recycled in the general process of depolymerization.

According to another embodiment, HCl is added to said purified alkaline solution to precipitate TPA as an acid; in this case the resulting solution contains water and NaCl.

Advantageously, the organic solvent used to extract the glycol monomer, e.g. ethylene glycol, from the lutarious solid obtained after evaporation of water, is selected from methanol, ethanol, isopropyl alcohol, ethyl acetate. In other words, the organic solvent used in this step is suitable to remove the glycol monomer and some water and some NaOH dissolved in water from the concentrated mixture previously obtained.

According to an embodiment, the solvent in the above obtained organic phase, containing solvent, EG, water and NaOH is evaporated to provide a liquid phase and NaOH. It is an advantage of the invention process that said NaOH can be recycled by using it in a further depolymerization reaction of blend textiles. Typically, the NaOH has a purity of 60-70%.

According to an embodiment, the above disclosed liquid phase is distilled to separate the solvent from a mixture of EG (or other glycole monomers) and water; advantageously said solvent is reused in a further extraction according to step b).

### BRIEF DESCRIPTION OF THE DRAWINGS

Further aspects and advantages in accordance with the present invention will be discussed more in detail with reference to the enclosed drawings, given by way of nonlimiting example, wherein:

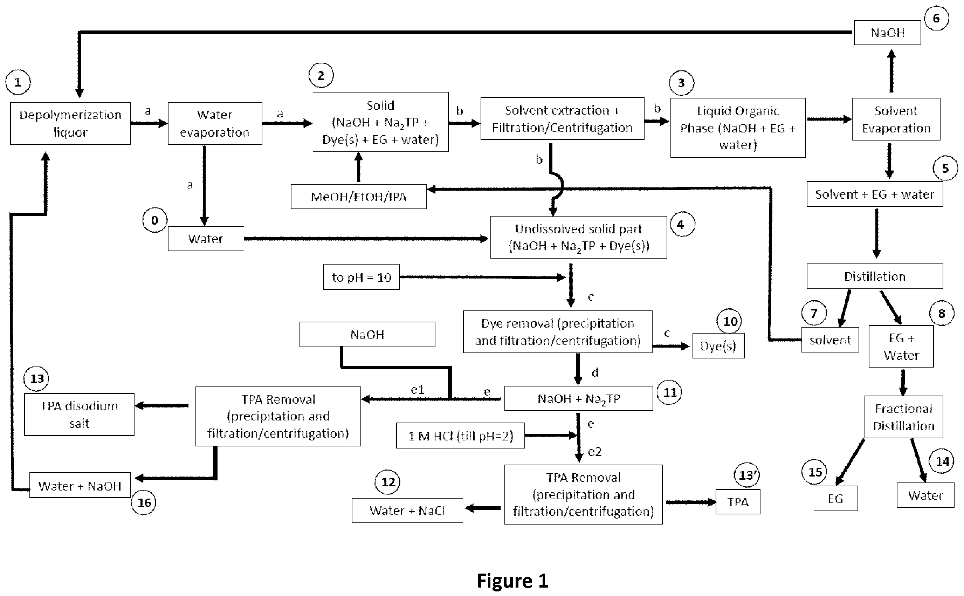

Figure 1 is a flow chart of an embodiment of the invention.

### DETAILED DISCLOSURE OF THE PRESENT INVENTION

As visible in the flow chart of figure 1, the process of recovering NaOH and polyester monomers resulting from an alkaline depolymerization reaction of polyester in a blend textile, comprises the steps of providing an aqueous alkaline depolymerization liquor 1 containing polyester monomers, NaOH and optionally dyes; evaporating water from said liquor to reach a lutarious, mud-like concentrated mixture 2 comprising water, solid NaOH, dyes and the solid and liquid products of the depolymerization reaction; extracting EG from said lutarious mixture with an organic solvent; modifying the pH of the solid phase remaining from the extraction of EG to first dissolve and subsequently precipitate TPA as a sodium salt or as an acid.

A depolymerization liquor 1 obtained from a process of alkaline depolymerization of a blend textile is initially provided; the liquor contains all the products and the reagents that were used in the process apart from the cellulosic fibers and non-polyester fibers of the textile. The liquor 1 is heated (step b) under vacuum, i.e. at a reduced pressure at a temperature below 100°C, preferably in the range of 50°C to 95°C, most preferably 75°C to 85°C. In an embodiment, a typical pressure value is in the range of 100 mmHg to 700 mmHg, preferably 250 mmHg to 700 mmHg. Water is evaporated from liquor 1 until the liquor becomes a concentrated lutarious mixture having a solid content of about 50% to 80% by weight and the consistency of mud. The evaporated water 0 is collected to be further used in the process.

The concentrated mixture 2 is extracted (step b) with an organic solvent to obtain a liquid organic phase 3 and a solid phase 4. The liquid phase contains the organic solvent and extracted ethylene glycol (EG), water and NaOH; the solid phase 4 contains NaOH, Na₂TPA, vat dyes, e.g. indigo and indigoid dyes, if they were present in the textile. The organic solvent is a polar solvent, preferably selected from methanol, ethanol, isopropyl alcohol, ethyl acetate and mixtures thereof. A preferred solvent is isopropanol.

Extraction is carried out preferably at room temperature. After extraction the solid portion is separated from the liquid phase, preferably by centrifugation e.g. at 4500 - 5000 rpm to provide an undissolved solid portion 4 and a liquid organic phase portion 3. In an embodiment, the undissolved solid portion 4 is dried, e.g. in an oven at 60-70°C to remove any organic solvent that may be present.

The liquid organic phase containing solvent, EG, water and NaOH is collected and evaporated under vacuum (preferably in the range 250 mmHg to 700 mmHg) at a temperature in the range of 50°C to 80°C, preferably 65°C to 75°C to remove EG, solvent and water. The remaining solid comprises NaOH, 6, with a purity of at least 60%, usually at least 70% by weight. The remaining part by weight typically comprises impurities such as polyester fragments or cellulose fragments not fully depolymerized. Thus the obtained NaOH may be purified. The solid NaOH may be used, without purification, to increase the NaOH content of the initial depolymerization liquor 1; additionally, it may also be used, without having to be purified, in a depolymerization reaction of blend textiles as previously disclosed with reference to EP23156251. Any impurity coming from this step will not affect the depolymerization process.

The liquid organic phase 5 comprising solvent 7, EG and water 8 is then distilled to separate the solvent 7 from EG and water 8; advantageously, the recovered solvent 7 is used in extraction step b), i.e. it is used again to extract the lutarious solid 2 obtained from step a) of the process. Preferably, the liquid phase 5 is distilled at a temperature in the range of 65 °C and 75 °C, preferably 82.5 °C to obtain solvent 7 and EG/water mixture 8. Ethylene glycol is recovered from mixture 8 in a way known per se in the art, e.g. by means of fractional distillation to separate EG 15 from water 14. Water 14 is advantageously reused in the process of the invention.

After extraction, the undissolved part, i.e. a mixture of Na₂TPA, NaOH, dyes, is treated, preferably at room temperature, with an aqueous alkaline solution having a pH low enough to dissolve NaOH and Na₂TPA; a suitable pH is in the range of 9.5 to 11.0, preferably a pH of about 10. In this condition, the insoluble vat dyes, in particular indigo and indigoid dyes, are separated and collected by filtration or by centrifugation (step d). The separated dyes 10 are washed several times with water and dried in an oven. The water used for washing the dyes is collected and reused in the process.

The purity of the thus obtained vat dyes is at least of 95%. The purity of the dyes is measured by spectroscopic methods (UV-Vis) means. The purified dyes are suitable to be reduced to a soluble, e.g. leuco, form to be used in a dyeing process.

In order to recover the terephthalic acid TPA from alkaline solution 11, containing NaOH and the disodium salt of TPA (Na₂TPA), the pH of said solution is modified in step e. Two possible ways of recovering TPA are provided.

In a first step (e2) TPA is precipitated in its acid form after adding an acid such as HCl, acetic acid, H₂SO₄, etc. until a pH of about 2 is reached; the precipitated TPA is separated, e.g. by filtration or centrifugation, and dried to give solid TPA 13'.

Alternatively, the process further comprises a step e1) wherein NaOH is added to said purified alkaline solution 11 to precipitate Na₂TPA. Na₂TPA precipitation is advantageously obtained by adding concentrated NaOH solution up to a NaOH concentration of 20 wt%.

Preferably the solid Na₂TPA 13 is separated from the said alkaline solution 11, e.g. it is filtered or centrifuged and washed several times with water. The remaining alkaline solution 16 contains water and NaOH and can be used in a depolymerization reaction of the blend textile.

The invention will now be further disclosed with reference to the following examples provided for illustrative and non-limiting purpose.

### EXAMPLE 1

2.5 L of a depolymerization solution (as obtained from a 25 consecutive cycles of a depolymerization process according to EP23156251) with a pH of 13.29 and a NaOH initial content of 3.06% by weight was subjected to vacuum evaporation at 90°C. This process yielded a slurry-like solid with a weight of 708 g and a solids content of 62%. A two-step extraction procedure using methanol and IPA was used to extract the desired components. The amount of solvent used was determined by the following calculation:

### Volume of solvent (ml) = Amount of solid (g) * 2.5

After extraction, the liquid and solid phases were separated by centrifugation at approximately 5000 rpm for 15 minutes. The organic solvent collected was 94% of the initial solvent volume, while the dried solid constituted 37% of the initial solid amount. The solvent was then evaporated under vacuum at 70°C, yielding 98 grams (82% yield) of NaOH (65% pure). The collected organic solvent (with a collection yield of 95%) was distilled at 82.5°C, which allowed the recovery of the pure organic solvent for reuse in the extraction process.

Following distillation, a mixture of water and EG was further fractionally distilled to separate 37.67 grams of EG. The solid residue obtained after extraction was dissolved in water with a pH of 10, resulting in the precipitation and collection of 4 grams of indigo dye by centrifugation.

Dyes purity was measured by spectroscopic methods (UV-Vis) means. The pH of the dissolved portion was adjusted to pH 2 with HCl, resulting in the precipitation and collection of 100.87 grams of TPA by centrifugation.

### EXAMPLE 2

After having being used for 30 cycles each in a process according to EP23156251, the depolymerization, bleaching and washing solutions were collected and mixed. From the mixture, 2200 ml of solution was taken and concentrated by evaporation under vacuum at 90°C to a slurry. After evaporation, 596 grams of solid with 50% solid content was obtained. Then the same procedure as described above in example 1 was carried out. The recovered amounts of TPA, EG, NaOH and indigo were 62.04 g of TPA, 22.7 g of EG, 58 g of NaOH (62% pure, 85% yield)and 2.4 g of indigo dye (in its solid, coloured form, 96% pure), respectively. In conclusion, the invention provides a process of recycling the liquor (i.e. the depolymerizing solution) resulting from a depolymerization of polyester filaments in a blend fabric to isolate and recover polyester monomers (TPA, Na₂TPA, EG and other glycols), NaOH and indigo or other vat dyes.

The invention also provides a process of recovering vat dyes, particularly indigo, from a waste textile. According to this embodiment, a textile, preferably a waste textile, is treated with an alkaline solution by passing a flow of said alkaline solution through said textile, wherein the textile is maintained in a static condition, to remove vat dyes from the textile and collecting, i.e. separating, the insoluble vat dyes from the resulting solution.

Typically, the alkaline solution is pumped through a container housing said waste textile and is circulated several times through said textile. The alkaline solution suitable for the vat dye, preferably indigo and indigoid dyes, removal process is an alkaline solution as above disclosed, in particular at paragraphs [004] to [006]. A suitable solution has a pH between 10 and 14. A preferred concentration of NaOH in the solution is in the range of 33g/L to 237 g/L of NaOH, preferably 36 g/L to 43 g/L of NaOH.

## Claims

1. A process of recovering NaOH, dyes polyester monomers resulting from an alkaline depolymerization reaction of polyester in a blend textile, comprising the steps of providing an aqueous alkaline depolymerization liquor (1) containing polyester monomers, NaOH and optionally dyes; concentrating said liquor to a mixture (2) comprising water, solid NaOH and the solid and liquid products of the depolymerization reaction; extracting EG from said mixture (2) with an organic solvent; modifying the pH of the solid phase (4) remaining from the extraction of EG to first dissolve and subsequently precipitate TPA as a sodium salt or as an acid.

2. The process of claim 1 comprising the following steps:
a) evaporating part of the water from said depolymerization liquor (1) to provide said concentrated mixture (2) containing reaction products of depolymerization;
b) extracting the said mixture (2) with an organic solvent to obtain a liquid phase (3) containing EG, water and NaOH and a solid phase (4) containing NaOH, Na₂TPA, optionally dyes;
c) dissolving at least the Na₂TPA of said solid portion (4) in an alkaline solution having pH in the range of 9.5 to 11.0 whereby in said solution is present a solid content (10) including dyes;
d) separating said solid content (10) including dyes from said alkaline solution to provide a purified alkaline solution (11) and solid dyes;
e) precipitating TPA from said purified alkaline solution (11).

3. The process of claim 2, further comprising step e1) wherein NaOH is added to said purified alkaline solution (11) to precipitate Na₂TPA (12).

4. The process of claim 2, further comprising a step e2) wherein HCl is added to said purified alkaline solution (11) to precipitate TPA (13').

5. The process of claim 3, wherein said solid Na₂TPA (13) is separated from said alkaline solution and said alkaline solution is used in a depolymerization reaction of blend textiles.

6. The process according to any previous claim, wherein said organic solvent is selected from methanol, ethanol, isopropyl alcohol, ethyl acetate and mixtures thereof.

7. The process according to any prevous claim, wherein said organic phase (3) containing solvent, EG, water and NaOH is evaporated to provide a liquid phase (5) and NaOH (6) and wherein said NaOH is used in a depolymerization reaction of blend textiles.

8. The process according to claim 7, wherein said liquid phase (5) is distilled to provide solvent (7) and a mixture of EG and water (8), said solvent being reused in extraction step b).

9. The process of any previous claim, wherein said water evaporated from the depolymerization liquor (1) is added to said solid portion (4) to dissolve Na₂TPA of said solid portion (4).

10. The process of any previous claim, wherein water is evaporated from said depolymerization liquor (1) under vacuum at a temperature below 100°C until the solids content of the resulting concentrated mixture (2) is in the range of 50 to 80% by weight.

11. The process of any claim 2 to 10, wherein said dyes are vat dyes and further comprising the step of reducing the dyes present in said solid content of step d) of claim 2 to a soluble form in a reaction solution and filtering any remaining solid from said solution, to recover said dyes.

12. A process of recovering vat dyes, particularly indigo, from a textile comprising the steps of: treating a textile with an alkaline solution by passing a flow of said alkaline solution through said textile, said textile being maintained in a static condition, whereby vat dyes in their insoluble form are removed from said textile; and separating said insoluble vat dyes from the resulting alkaline solution.

13. The vat dye recovering process of claim 12, wherein said textile comprises polyester filaments, further comprising the step of depolymerizing said polyester into corresponding polyester monomers and separating the said monomers from said insoluble dyes.

14. The vat dye recovering process of claim 12 or 13, wherein said alkaline solution is pumped through a container housing said waste textile and is circulated several times through said textile.
